# FASCICULE DE BREVET EUROPEEN

(11) **EP 1 420 849 B1**
(45) Date de publication et mention de la délivrance du brevet: **31.05.2006**
(21) Numéro de dépôt: 03717384.6
(22) Date de dépôt: 07.02.2003
(51) Int. Cl.: A61M 25/06

(54) **ENSEMBLE MEDICAL COMPRENANT UN CATHETER, UNE AIGUILLE ET SON ETUI**
MEDIZINISCHES SET, DAS EINEN KATHETER, EINE NADEL UND IHREN BEHÄLTER ENTHÄLT
MEDICAL KIT COMPRISING A CATHETER, A NEEDLE AND ITS CASE

(30) Priorité: 08.02.2002 FR 0201600; 05.02.2003 FR 0301313
(43) Date de publication de la demande: 26.05.2004
(73) Titulaire: VYGON, F-95440 Ecouen (FR)
(72) Inventeur: ROSSI, Daniel, F-95630 Meriel (FR); HUET, Jean-Max, F-92110 Clichy (FR)
(74) Mandataire: Schrimpf, Robert
(86) Numéro de dépôt international: PCT/FR2003/000393
(87) Numéro de publication internationale: WO 2003/066151

(56) Documents cités:
- WO-A-00/02614
- WO-A-95/19193
- FR-A- 2 684 006
- US-A- 5 125 414
- US-A- 5 951 529
- US-A- 6 077 244
- US-B1- 6 193 690

## Description

L'invention concerne un ensemble médical comprenant un cathéter court, une aiguille de ponction enfilée dans le cathéter pour permettre l'introduction du cathéter dans le corps et un étui tubulaire raccordé de façon détachable au cathéter pour loger intégralement l'aiguille après la ponction et le retrait de l'aiguille hors du cathéter.

On connaît de nombreuses réalisations d'un tel ensemble (US 5 273 540, US 6 077 244, US 6 193 690, US 5 879 331, WO 95/19193).

Les publications US 5 879 331 et WO 95/19193 décrivent des réalisations dans lesquelles l'étui présente une fente longitudinale entre une sortie distale de raccordement au cathéter et une extrémité proximale opposée et porte un curseur monté à coulisse le long de la fente, ce curseur présentant une partie interne située dans l'étui et qui constitue une embase pour l'aiguille et une partie externe située sur l'étui et qui permet de solliciter manuellement le curseur pour le faire coulisser d'une position distale où l'aiguille traverse le cathéter jusqu'à une position proximale dans laquelle l'aiguille est totalement retirée du cathéter et rentrée dans l'étui.

Le problème se pose d'éviter que l'aiguille ainsi escamotée dans l'étui puisse ressortir de cet étui par la sortie de raccordement, après séparation de l'étui et du cathéter, sous l'effet d'une manipulation intempestive du curseur, et différentes solutions ont été proposées à cette fin.

Dans la solution décrite dans la publication US 5 879 331, l'étui est muni d'un bras mobile qui lorsqu'il est contacté par le curseur bascule et vient solliciter l'aiguille près de son extrémité de ponction pour incliner l'aiguille en sorte qu'elle ne soit plus axée sur la sortie de raccordement au cathéter.

Dans une réalisation décrite dans la publication WO95/19193 l'étui est conformé intérieurement, à proximité de la fin de la course du curseur vers ladite extrémité proximale de l'étui, en sorte que le contact du curseur avec l'étui provoque en fin de course une inclinaison du curseur et par conséquent de l'aiguille dans l'étui.

La présente invention vise à assurer une sécurité anti-pique tout en simplifiant le dispositif et en diminuant son encombrement extérieur.

On y parvient selon l'invention, en prévoyant un déviateur fixe (13 ; 16 ; 22) disposé dans l'étui sur le trajet du curseur à proximité de la fin de la course du curseur vers ladite extrémité proximale (3b) de l'étui, en sorte que le contact du curseur avec le déviateur provoque une inclinaison du curseur et par conséquent de l'aiguille dans l'étui, et des moyens de rappel conçus pour repousser ensuite le curseur incliné vers l'extrémité distale de l'étui en sorte que la pointe de l'aiguille se trouve immobilisée.

Ces moyens de rappel sont constitués de préférence par des moyens prévus sur le curseur et des moyens prévus sur l'étui, ces moyens étant disposés pour entrer en contact en fin de course du curseur vers ladite extrémité proximale et coopérant pour que le curseur soit repoussé en sens inverse par un effet élastique.

De préférence, la pointe de l'aiguille inclinée se trouve repoussée jusqu'à se planter dans la paroi de l'étui ou dans la paroi d'un raccord monté à l'extrémité distale de l'étui.

De préférence, la pointe de l'aiguille inclinée et repoussée est reçue dans une gorge réalisée autour de la sortie d'aiguille de l'étui.

On décrira ci-après à titre d'exemple, des réalisations non limitatives d'un ensemble conforme à l'invention, en référence aux figures du dessin joint sur lequel :
- la figure 1 est un schéma des différents états d'une première réalisation-au cours de phases successives A à F de la rentrée de l'aiguille dans l'étui tubulaire ;
- la figure 2 est un schéma agrandi de l'extrémité proximale de l'étui tubulaire au cours des phases D, E et F de la figure 1 ;
- la figure 3 est un schéma des différents états d'une deuxième réalisation au cours de phases successives A à F de la rentrée de l'aiguille dans l'étui tubulaire ;
- la figure 4 est un schéma agrandi de l'extrémité proximale de l'étui tubulaire au cours des phases D, E et F de la figure 3 ;
- la figure 5 est un schéma des différents états d'une troisième réalisation au cours de phases successives A à F de la rentrée de l'aiguille dans l'étui tubulaire ;

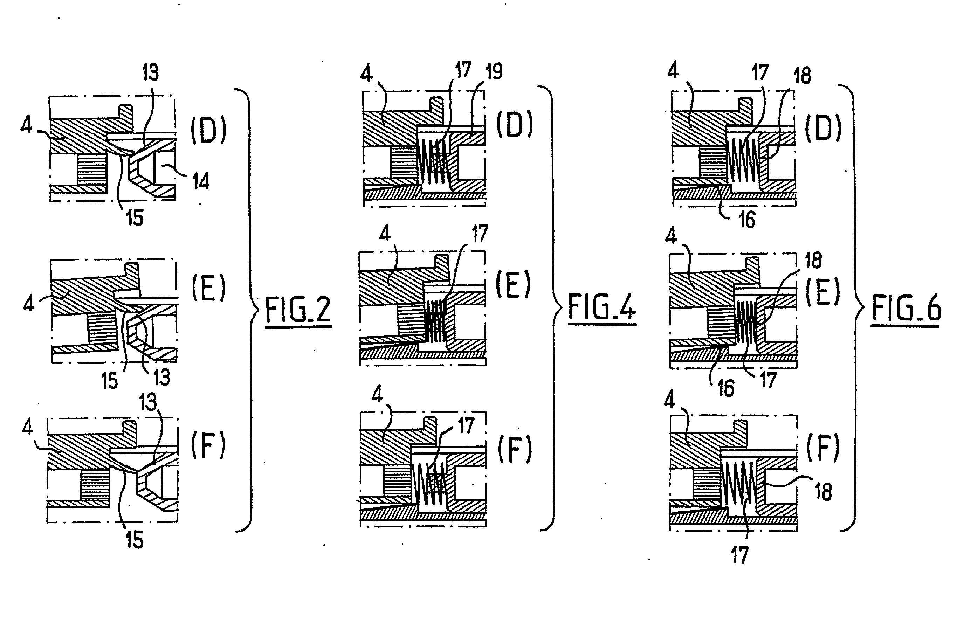

- la figure 6 est un schéma agrandi de l'extrémité proximale de l'étui tubulaire au cours des phases D, E et F de la figure 5 ;
- la figure 7 est un schéma des phases successives de la mise en oeuvre d'un ensemble selon l'invention ;
- la figure 8 est un schéma comparable à celui de la figure 7 pour une variante du processus de mise en oeuvre ;
- la figure 9 est un schéma comparable à celui de la figure 7 pour une autre variante de mise en oeuvre ;
- la figure 10 est une section droite courante de l'étui dans un plan X-X ;
- la figure 11 est une coupe axiale agrandie de l'extrémité proximale de l'étui de la figure 1 avec son bouchon qui est représenté séparé sur la figure pour la clarté de la figure ;
- la figure 12 est une coupe axiale agrandie de l'extrémité distale de l'étui d'une réalisation selon la figure 1F, 3F ou 5F,
- la figure 13 est une section droite d'un curseur et d'un étui selon l'invention, et
- la figure 14 est un schéma des différents états d'une quatrième réalisation au cours de phases successives A à D de la rentrée de l'aiguille dans l'étui tubulaire.

L'ensemble représenté sur les figures comprend un cathéter court (1), une aiguille de ponction (2) pour permettre l'introduction du cathéter dans une veine, un étui cylindrique (3) raccordé de façon détachable au cathéter et un curseur (4) monté à coulisse sur l'étui pour faire rentrer l'aiguille dans le tube après la ponction.

L'étui (3) a une extrémité distale (3a) agencée ou munie d'un raccord (19) pour son raccordement au cathéter, une extrémité proximale opposée (3b) et une fente latérale longitudinale (3c) située entre ces extrémités. L'étui porte un curseur (4) monté à coulisse le long de la fente et qui présente une partie externe (4a) située sur l'étui et une partie interne (4b) située dans l'étui, ces deux parties se raccordant l'une à l'autre à travers la fente de l'étui.

La partie externe (4a) est conçue pour permettre de solliciter le curseur afin de le faire coulisser le long de la fente.

La partie interne (4b) est fixée à l'aiguille dont elle constitue une embase creuse fermée par un filtre air-vent (5) et équipée d'une valve qui s'ouvre sous la pression d'injection d'une seringue.

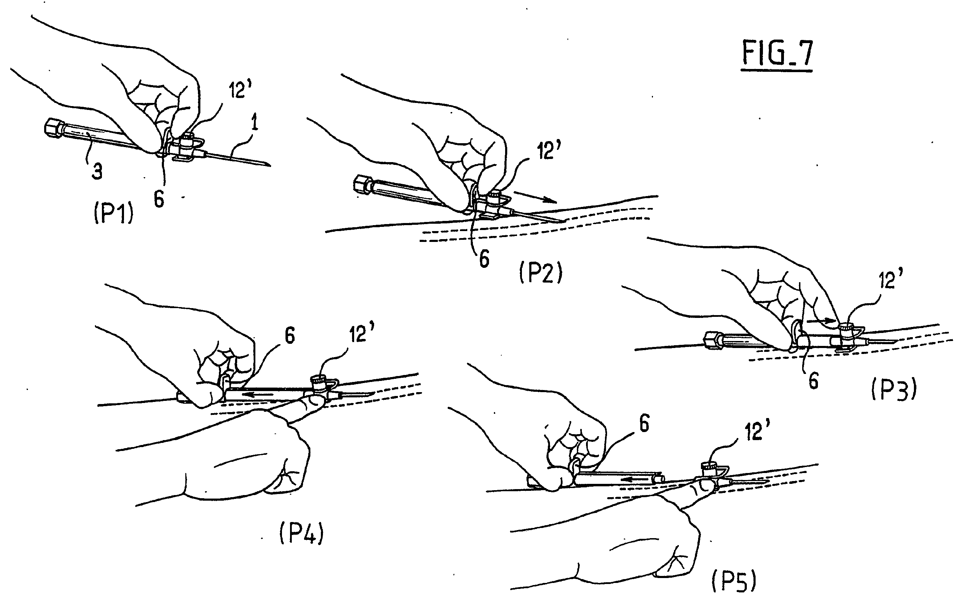

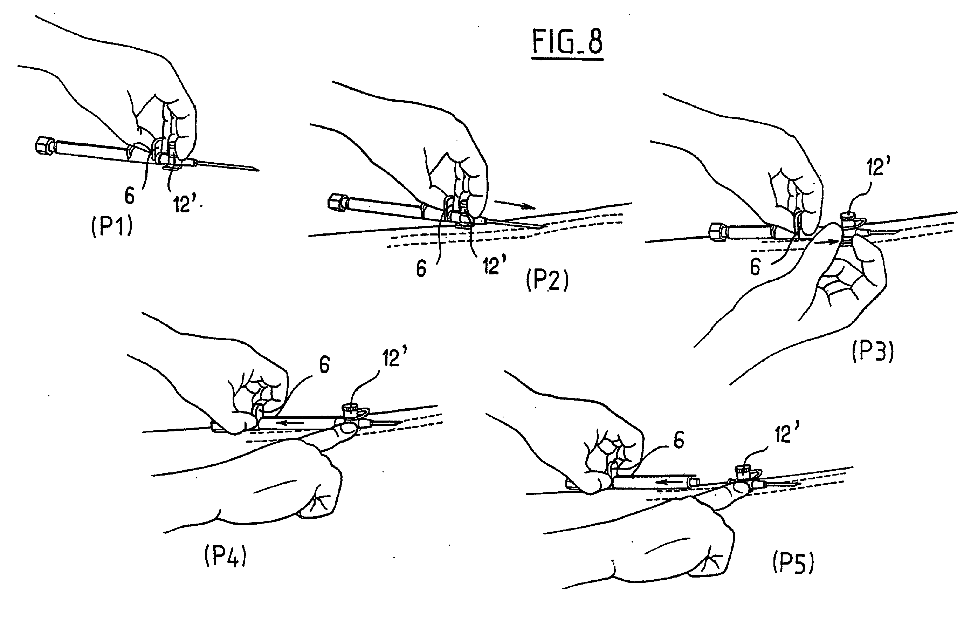

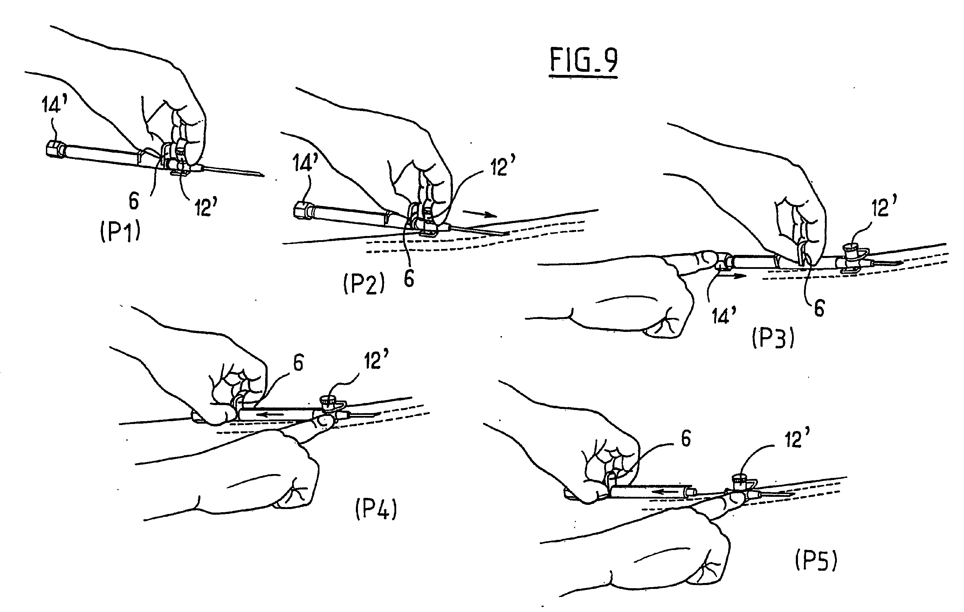

Dans les exemples illustrés, la partie externe (4a) du curseur présenté un plateau transversal (6) que le manipulateur peut solliciter de différentes manières pour effectuer la ponction et ensuite pour retirer l'aiguille, comme on le voit sur les figures 7 à 9.

Le cathéter est muni d'un raccord embase (7) de type en soi connu et l'extrémité distale (3a) de l'étui est conçue pour pénétrer dans ce raccord ou est munie d'un raccord adéquat (19).

Le raccord du cathéter présente un bourrelet extérieur (8) placé à l'arrière du raccord et l'extrémité distale (3a) de l'étui ou le raccord (19) de l'étui présente une languette (9) dirigée vers l'avant, qui se positionne sur le raccord en avant du bourrelet lorsque l'étui est raccordé au cathéter, mais qui est suffisamment flexible pour passer à force sur le bourrelet lorsque l'étui est séparé du raccord.

L'extrémité distale ou le raccord distal (19) de l'étui est normalement fermée par une cloison transversale (10') qui permet le passage axial de l'aiguille, par exemple grâce à un trou (11).

Le raccord (7) du cathéter peut présenter une entrée latérale (12) de façon en soi connue, pour permettre des injections. Cette entrée (12) est fermée par un capuchon (12') .

L'étui tubulaire comporte des moyens déviateurs fixes pour désaxer l'aiguille après que celle-ci soit rentrée dans l'étui, afin d'éviter que l'aiguille puisse ressortir par l'extrémité distale de l'étui sous l'effet d'une sollicitation intempestive du curseur.

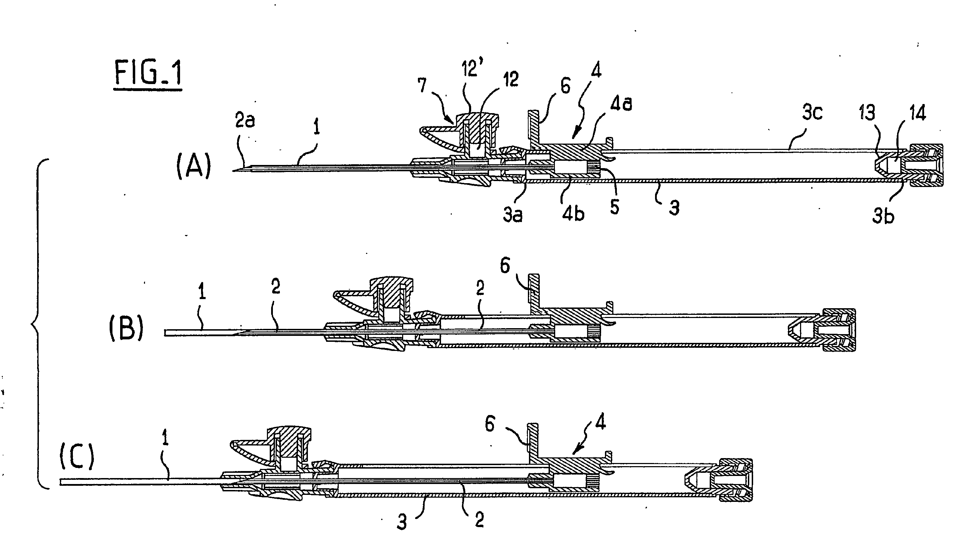

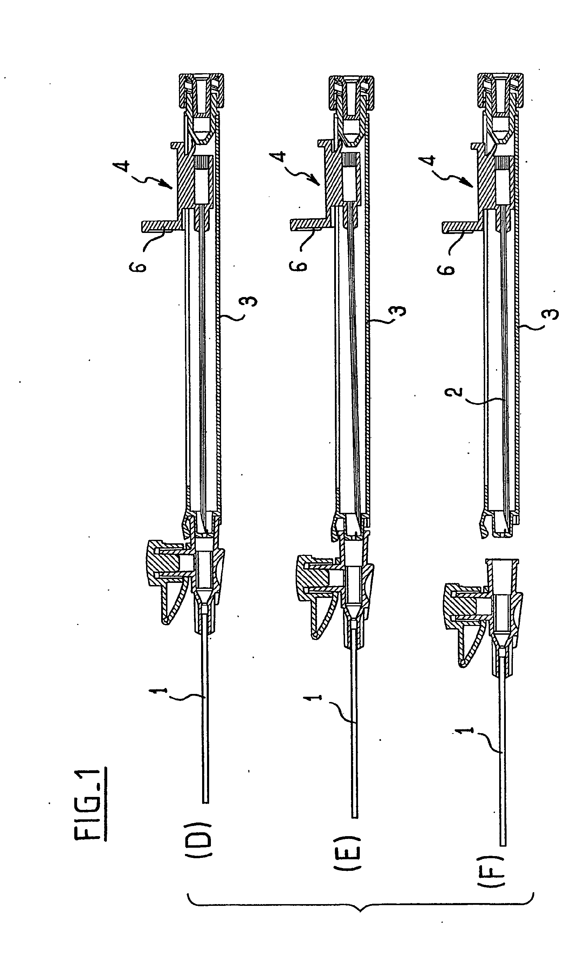

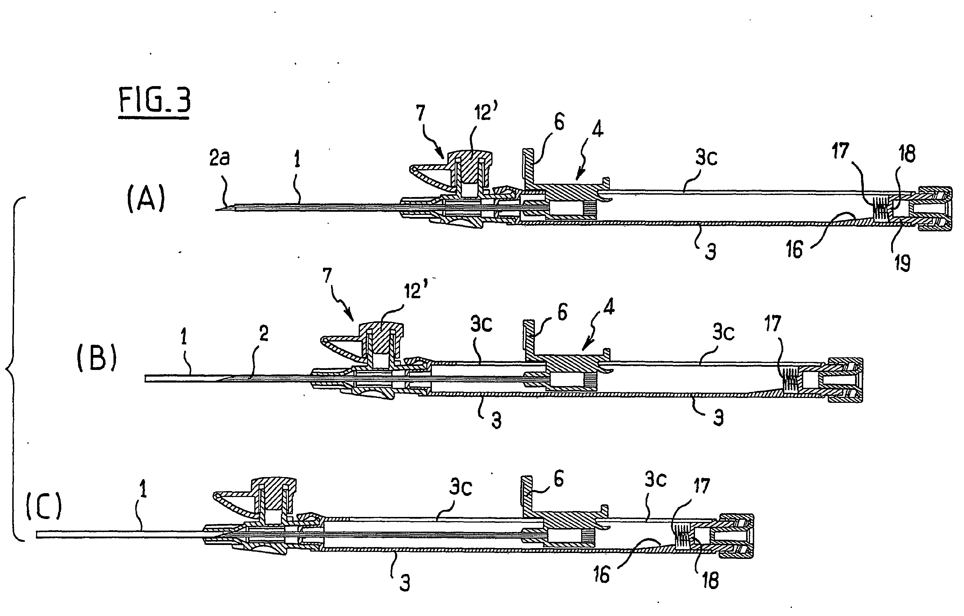

Dans la réalisation des figures 1 et 2, ces moyens déviateurs sont constitués par une rampe (13) formée sur une pièce (14) enfoncée dans l'extrémité proximale (3b) de l'étui et bloquée par un bouchon (14'). Cette pièce a par exemple une forme tronconique, la paroi conique constituant une rampe. Le curseur est muni dans sa partie interne d'une languette élastique (15) dirigée vers l'arrière et dont le contact avec la rampe provoque une déviation du curseur, ce qui désaxe l'aiguille (figures 1D, 1E) au fur et à mesure que le curseur monte la rampe.

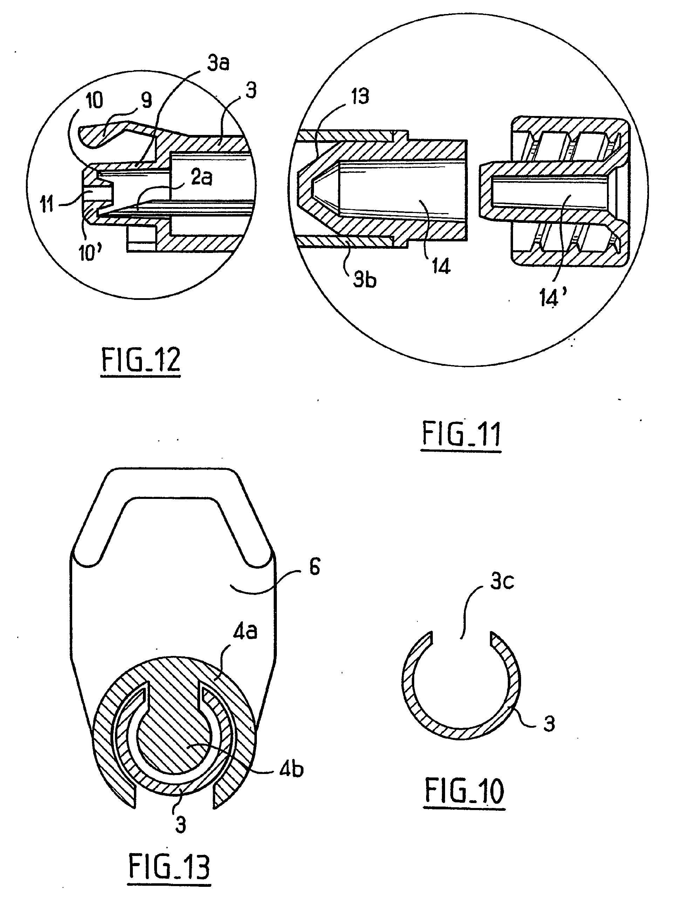

En fin de course, le curseur est rappelé en sens inverse par l'effet de la languette en sorte que la pointe biseautée de l'aiguille (2a) inclinée vient se loger dans une gorge cylindrique (10) ménagée à l'intérieur de l'extrémité distale (3a) de l'étui autour de la sortie (11) jusqu'à se planter contre la paroi (10') à l'avant de l'étui, ce qui bloque l'aiguille (figure 1F et figure 12).

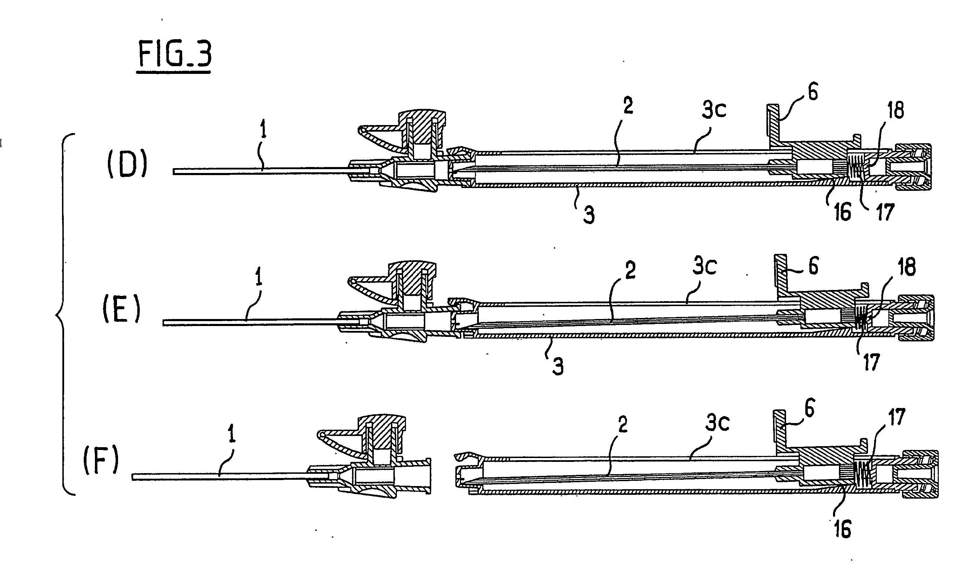

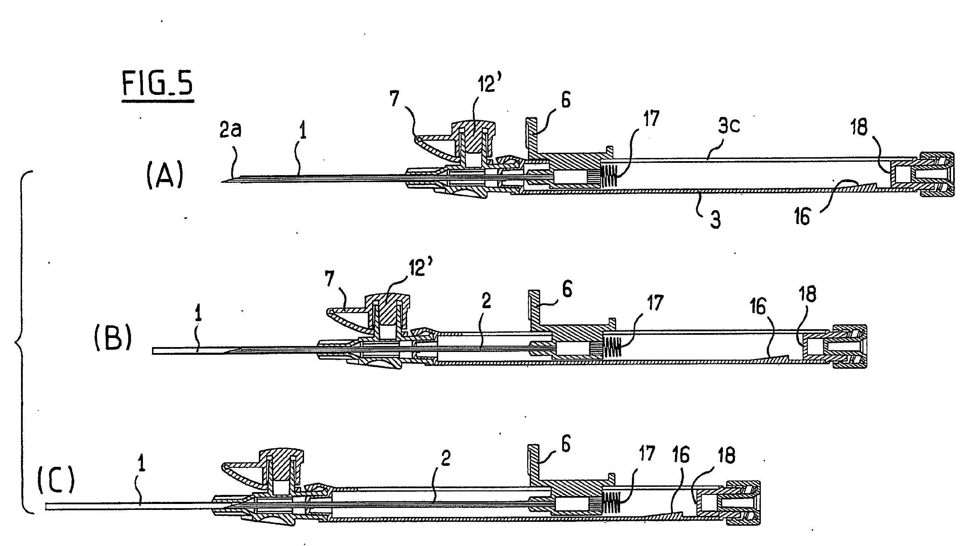

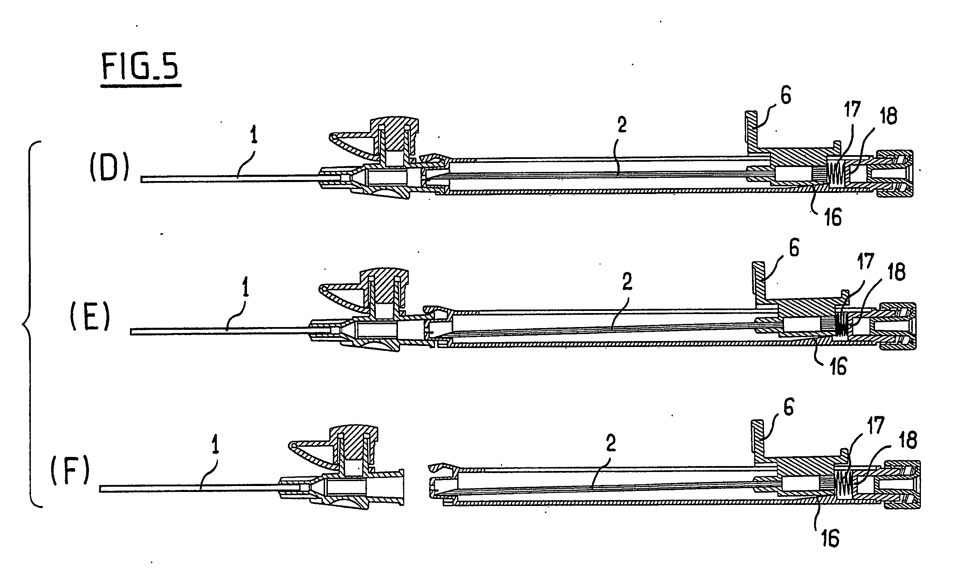

Dans la réalisation des figures 3 et 5, une rampe (16) est formée par un bossage interne de la paroi latérale de l'étui à proximité de l'extrémité proximale (3b) de l'étui et disposée sur le parcours de la partie interne (4b) du curseur en sorte que ce bossage provoque une inclinaison du curseur lorsque celui-ci passe sur le bossage (figures 3D, 3E, 5D, 5E) . Un ressort hélicoïdal (17) disposé entre la partie interne du curseur et une butée (18) située dans l'extrémité proximale (3b) de l'étui repousse ensuite le curseur dans une mesure suffisante pour que la pointe biseautée de l'aiguille vienne se fixer dans la paroi (10') située à l'avant de l'étui (figures 3F, 5F et 12).

Le ressort (17) est fixé à la butée (18), comme dans la réalisation de la figure 3 ou il est fixé à la partie interne du curseur comme dans la réalisation de la figure 5.

La butée (18) est constituée par la face avant d'une pièce (18') introduite comme un bouchon dans l'extrémité proximale de l'étui.

Les figures 7 à 9 montrent des phases successives (P1 à P5) d'un processus de mise en place d'un cathéter au cours desquelles l'opérateur se sert d'une main ou des deux mains pour agir sur le plateau (6) du curseur, le capuchon (12') du raccord ou le bouchon (14') pour ponctionner (P1,P2) retirer progressivement l'aiguille (P3,P4) et détacher du raccord de cathéter l'étui contenant l'aiguille (P5).

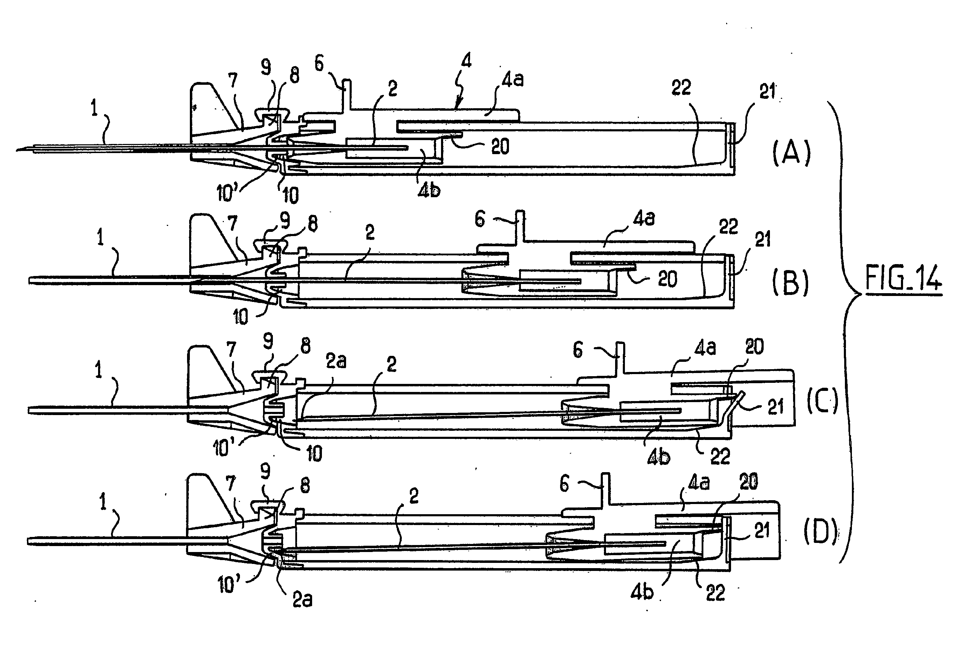

Dans la réalisation de la figure 14, les moyens de rappel de l'aiguille inclinée vers l'extrémité distale de l'étui comprennent un ergot rigide (20) en saillie vers l'extrémité proximale de l'étui, sur la partie interne (4b) du curseur (4) qui constitue l'embase de l'aiguille, et une lame élastique (21) disposée dans le travers de l'extrémité proximale (3b) de l'étui (3) en sorte que cette lame, sollicitée par l'ergot, après déviation de l'aiguille (figure 14C) et relâchement de la traction de l'utilisateur sur le curseur, repousse en sens inverse le curseur dans une mesure suffisante pour que la pointe de l'aiguille se loge dans une paroi de l'extrémité distale de l'étui ou du raccord (19) présent à cette extrémité (figure 14D). Par exemple, elle est bloquée dans la paroi de fond d'une gorge cylindrique (10) formée dans le raccord monté à l'extrémité distale de l'étui pour le raccordement du cathéter.

De préférence, comme dans le cas représenté, la lame élastique est constituée par une paroi venue de moulage avec la paroi de l'étui mais qui n'est solidaire de la paroi latérale de l'étui que sur une partie de son pourtour.

Dans la réalisation de la figure 14, les moyens déviateurs sont constitués par une rampe (22) venue de moulage d'injection avec la paroi longitudinale de l'étui (comme la rampe 16 de la réalisation des figures 3 et 5) et avec la lame élastique (21) laquelle solidaire de l'extrémité de cette rampe et n'a pas d'autres liaisons avec le tube.

On notera que cette réalisation est particulièrement simple.

L'invention n'est pas limitée aux réalisations qui ont été décrites.

## Revendications

1. Ensemble médical comportant un cathéter court (1), une aiguille de ponction (2) enfilée dans le cathéter pour permettre l'introduction du cathéter dans le corps et un étui tubulaire (3) raccordé de façon détachable au cathéter pour loger intégralement l'aiguille après la ponction et le retrait de l'aiguille hors du cathéter, l'étui présentant une fente longitudinale (3c) entre une extrémité de sortie distale (3a) de raccordement au cathéter et une extrémité proximale opposée (3b) et portant un curseur (4) monté à coulisse le long de la fente pour commander ledit retrait de l'aiguille, ce curseur présentant une partie interne (4b) située dans l'étui et qui constitue une embase pour l'aiguille et une partie externe (4a) située sur l'étui et qui permet de solliciter manuellement le curseur pour le faire coulisser en direction de ladite extrémité proximale jusqu'à une position proximale dans laquelle l'aiguille est totalement rentrée dans le tube, l'étui comportant un déviateur fixe (13 ; 16 ; 22) disposé dans l'étui sur le trajet du curseur à proximité de la fin de la course du curseur vers ladite extrémité proximale (3b) de l'étui, en sorte que le contact du curseur avec le déviateur provoque une inclinaison du curseur et par conséquent de l'aiguille dans l'étui, **caractérisé en ce que** des moyens de rappel (15, 13 ; 17 ; 20, 21) sont prévus pour repousser en sens inverse le curseur incliné, en sorte que la pointe (2a) de l'aiguille inclinée se trouve immobilisée.

2. Ensemble selon la revendication 1, dans lequel l'extrémité distale (3a) de l'étui présente une gorge (10) réalisée autour d'une sortie d'aiguille (11) de l'étui pour recevoir la pointe de l'aiguille inclinée.

3. Ensemble selon la revendication 2 dont la gorge (10) est formée dans un raccord (19) disposé dans cette extrémité distale pour le raccordement du cathéter.

4. Ensemble selon l'une des revendications 1 à 3, dans lequel le déviateur est constitué par une pièce (14) disposée dans l'extrémité proximale de l'étui (3b).

5. Ensemble selon l'une des revendications 1 à 3, dans lequel ledit déviateur est constitué par une pièce (14) enfilée et maintenue dans l'extrémité proximale de l'étui et qui présente une rampe (13) sur laquelle monte la partie interne du curseur.

6. Ensemble selon la revendication 5, dans lequel la partie interne du curseur présente une languette élastique (15) qui frotte sur cette rampe (13) et qui agit en fin de course pour repousser le curseur incliné.

7. Ensemble selon l'une des revendications 1 à 3, dans lequel le déviateur est une rampe (16) constituée par un bossage interne de la paroi latérale de l'étui.

8. Ensemble selon la revendication 7, et qui comporte un ressort hélicoïdal (17) disposé entre la partie interne (4b) du curseur et une butée (18) disposée dans l'extrémité proximale (3b) de l'étui pour repousser en fin de course le curseur incliné.

9. Ensemble selon la revendication 8, dans lequel ledit ressort (17) est fixé à la partie interne (4b) du curseur.

10. Ensemble selon la revendication 8 dans lequel ledit ressort (17) est fixé à ladite butée (18).

11. Ensemble selon l'une des revendications 8 à 9, dans lequel la butée (18) est la face avant d'un bouchon (19) introduit dans l'extrémité proximale (3b) de l' étui .

12. Ensemble selon la revendication 1 dans lequel lesdits moyens de rappel sont constitués par un ergot rigide (20) en saillie vers l'extrémité proximale de l'étui sur la partie interne (4b) du curseur (4) et par une lame élastique (21) disposée à l'extrémité proximale de l'étui en sorte que cette lame, sollicitée par l'ergot après inclinaison de l'aiguille, repousse en sens inverse le curseur et l'aiguille dans une mesure suffisante pour que la pointe (2a) de l'aiguille inclinée soit bloquée dans l'extrémité distale (3a) de l'étui.

13. Ensemble selon la revendication 12 dans lequel ladite extrémité distale (3a) de l'étui est munie d'un raccord (19) pour le montage du cathéter et dans lequel ledit raccord (19) présente autour de la sortie d'aiguille (11) de l'étui une gorge (10) pour recevoir la pointe (2a) de l'aiguille inclinée et repoussée.

14. Ensemble selon la revendication 12 ou 13 dans lequel ladite lame élastique (21) est formée par une paroi située dans le travers de l'extrémité proximale de l'étui mais qui n'est solidaire de l'étui que sur une partie de son pourtour.

15. Ensemble selon l'une des revendications 12 à 14 dans lequel l'étui comporte un moyen déviateur constitué par une rampe (22) constituée par un bossage interne de la paroi latérale de l'étui et dans lequel ladite paroi qui constitue la lame élastique (21) est venue de moulage par injection à l'extrémité de cette rampe.

16. Ensemble selon l'une des revendications 1 à 15 dans lequel le cathéter court est muni d'un raccord d'entrée (7) qui sert d'appui de poussée pour l'utilisateur et dans lequel l'extrémité distale (3a) de raccordement de l'étui (3) au cathéter est conformée pour pénétrer dans ce raccord et pour l'accrochage de cette extrémité sur le raccord.

17. Ensemble selon la revendication 16 dans lequel l'extrémité distale de l'étui est pourvue d'un raccord (19) pour son raccordement au cathéter, lequel raccord pénètre dans le raccord du cathéter.

## Claims

1. A medical assembly comprising a short catheter (1), a puncturing needle (2) engaged in the catheter to enable the catheter to be inserted into a body, and a tubular sheath (3) detachably coupled to the catheter to receive the entire needle after the needle has been used for puncturing and once it has been withdrawn from the catheter, the sheath presenting a longitudinal slot (3c) between a distal outlet end (3a) for coupling to the catheter and an opposite proximal end (3b), and carrying a slider (4) mounted to slide along the slot to control said withdrawal of the needle, the slider presenting an internal portion (4b) situated inside the sheath and constituting a base for the needle, and an external portion (4a) situated on the sheath and enabling the slider to be pushed manually to cause to it to slide towards said proximal end to a proximal position in which the needle is fully withdrawn into the tube, the sheath comprising a stationary deflector (13; 16; 22) disposed inside the sheath on the path of the slider close to the end of the stroke of the slider towards said proximal end (3b) of the sheath, so that contact between the slider and the deflector causes the slider to be inclined and consequently causes the needle to be inclined inside the sheath, the assembly being **characterized in that** return means (15, 13; 17; 20, 21) are provided so as to push the inclined slider back in the opposite direction so that the point (2a) of the inclined need is prevented from moving.

2. An assembly according to claim 1, in which the distal end (3a) of the sheath presents a groove (10) made around a needle outlet (11) of the sheath to receive the tip of the inclined needle.

3. An assembly according to claim 2, having a groove (10) that is formed in a coupling (19) disposed in said distal end for connecting the catheter.

4. An assembly according to any one of claims 1 to 3, in which the deflector is constituted by a piece (14) disposed in the proximal end (3b) of the sheath.

5. An assembly according to any one of claims 1 to 3, in which said deflector is constituted by a piece (14) engaged and held in the proximal end of the sheath and presents a ramp (13) on which an internal portion of the slider becomes engaged.

6. An assembly according to claim 5, in which the internal portion of the slider presents a resilient tongue (15) which rubs against said ramp (13) and which acts at the end of the slider stroke to push back the inclined slider.

7. An assembly according to any one of claims 1 to 3, in which the deflector is a ramp (16) constituted by an internal projection from the side wall of the sheath.

8. An assembly according to claim 7, and including a helical spring (17) disposed between the internal portion (4b) of the slider and an abutment (18) disposed in the proximal end (3b) of the sheath to push the inclined slider back at the end of its stroke.

9. An assembly according to claim 8, in which said spring (17) is fixed to the internal portion (4b) of the slider.

10. An assembly according to claim 8, in which said spring (17) is fixed to said abutment (18).

11. An assembly according to claim 8 or claim 9, in which the abutment (18) is the front face of a stopper (19) inserted in the proximal end (3b) of the sheath.

12. An assembly according to claim 1, in which said return means are constituted by a rigid lug (20) projecting towards the proximal end of the sheath over the inner portion (4b) of the slider (4), and by a resilient blade (21) disposed at the proximal end of the sheath so that said blade, pushed by the lug, after inclination of the needle, pushes the slider and the needle back in the opposite direction over a distance that is sufficient for the point (2a) of the inclined needle to be blocked in the distal end (3a) of the sheath.

13. An assembly according to claim 12, in which said distal end (3a) of the sheath is provided with a coupling (19) for mounting the catheter, and in which said coupling (19) presents, around the needle outlet (11) of the sheath, a groove (10) for receiving the point (2a) of the inclined and pushed-back needle.

14. An assembly according to claim 12 or claim 13, in which said resilient blade (21) is formed by a wall situated in the cross-piece of the proximal end of the sheath, but which is secured to the sheath only over a portion of its circumference.

15. An assembly according to any one of claims 12 to 14, in which the sheath comprises deflector means constituted by a ramp (22) constituted by an inside bulge of the side wall of the sheath, and in which said wall which constitutes the resilient blade (21) is injection molded integrally with the end of said ramp.

16. An assembly according to any one of claims 1 to 15, in which the short catheter is provided with an inlet coupling (7) which serves as a thrust bearing point for the user and in which the distal end (3a) of the coupling of the sheath (3) to the catheter is shaped in order to penetrate into said coupling and to secure said end to the coupling.

17. An assembly according to claim 16, in which the distal end of the sheath is provided with a coupling (19) for connecting it to the catheter, which coupling penetrates into the catheter coupling.

## Patentansprüche

1. Medizinisches Set, das folgendes umfaßt: einen Kurzkatheter (1), eine Punktionsnadel (2), die im Katheter geführt ist, um die Einführung des Katheters in den Körper zu gestatten, und einen röhrenförmigen Behälter (3), der abnehmbar mit dem Katheter verbunden ist, zur vollständigen Aufnahme der Nadel nach der Punktion und dem Zurückziehen der Nadel aus dem Katheter, wobei der Behälter zwischen einem distalen Austrittsende (3a) zum Anschluß des Katheters und einem entgegengesetzten proximalen Ende (3b) einen Längsschlitz (3c) hat und einen Schieber (4) trägt, der entlang des Schlitzes gleitend verschiebbar angebracht ist, um das Zurückziehen der Nadel zu betätigen, wobei dieser Schieber einen inneren Abschnitt (4b) hat, der sich im Behälter befindet und der für die Nadel ein Aufnahmeteil bildet, und einen äußeren Abschnitt (4a) hat, der sich auf dem Behälter befindet und der es gestattet, den Schieber manuell zu betätigen, um ihn in Richtung des proximalen Endes zu verschieben, bis zu einer proximalen Position, in der die Nadel vollständig in das Röhrchen eingezogen ist, wobei der Behälter einen festen Ablenker (13; 16; 22) umfaßt, der im Behälter, in der Bahn des Schiebers, in der Nähe des Endes des Schieberwegs in Richtung des proximalen Endes (3b) des Behälters angeordnet ist, derart, daß der Kontakt des Schiebers mit dem Ablenker eine Schrägstellung des Schiebers und **dadurch** der Nadel im Behälter bewirkt, **dadurch gekennzeichnet, daß** Rückholmittel (15, 13; 17; 20, 21) vorgesehen sind, um den schräggestellten Schieber in die entgegengesetzte Richtung zurückzudrücken, derart, daß die Spitze (2a) der schräggestellten Nadel festgestellt wird.

2. Set nach Anspruch 1, bei dem das distale Ende (3a) des Behälters eine um einen Nadelausgang (11) des Behälters herum ausgeführte Rille (10) aufweist, um die Spitze der schräggestellten Nadel aufzunehmen.

3. Set nach Anspruch 2, dessen Rille (10) in einem Anschlußteil (19) ausgebildet ist, das zum Anschluß des Katheters an diesem distalen Ende angeordnet ist.

4. Set nach einem der Ansprüche 1 bis 3, bei dem der Ablenker durch ein Teil (14) gebildet wird, das am proximalen Ende des Behälters (3b) angeordnet ist.

5. Set nach einem der Ansprüche 1 bis 3, bei dem der Ablenker durch ein Teil (14) gebildet wird, das in das proximale Ende des Behälters eingeführt ist und in diesem gehalten wird und das eine Rampe (13) hat, auf der der innere Abschnitt des Schiebers raufläuft.

6. Set nach Anspruch 5, bei dem der innere Abschnitt des Schiebers eine elastische Zunge (15) umfaßt, die auf der Rampe (13) reibt und die am Ende des Wegs wirksam wird, um den schräggestellten Schieber zurückzudrücken.

7. Set nach einem der Ansprüche 1 bis 3, bei dem der Ablenker eine Rampe (16) ist, die durch einen innenliegenden Höcker der Behälterseitenwand gebildet wird.

8. Set nach Anspruch 7, das eine Spiralfeder (17) umfaßt, die zwischen dem inneren Abschnitt (4b) des Schiebers und einem am proximalen Ende (3b) des Behälters angeordneten Anschlag (18) angeordnet ist, um am Wegende den schräggestellten Schieber zurückzudrücken.

9. Set nach Anspruch 8, bei dem die Feder (17) am inneren Abschnitt (4b) des Schiebers befestigt ist.

10. Set nach Anspruch 8, bei dem die Feder (17) am Anschlag (18) befestigt ist.

11. Set nach einem der Ansprüche 8 bis 9, bei dem der Anschlag (18) die Vorderseite eines Verschlusses (19) ist, der in das proximale Ende (3b) des Behälters eingeführt ist.

12. Set nach Anspruch 1, bei dem die Rückholmittel durch folgendes gebildet werden: eine steife Nase (20), die vom inneren Abschnitt (4b) des Schiebers (4) in Richtung des proximalen Behälterendes hervorsteht, und ein elastisches Plättchen (21), das am proximalen Ende des Behälters so angeordnet ist, daß dieses nach dem Schrägstellen der Nadel von der Nase betätigte Plättchen den Schieber und die Nadel in entgegengesetzter Richtung in ausreichendem Maße zurückdrückt, damit die Spitze (2a) der schräggestellten Nadel im distalen Ende (3a) des Behälters blockiert wird.

13. Set nach Anspruch 12, bei dem das distale Ende (3a) des Behälters mit einem Anschlußteil (19) zum Anbringen des Katheters ausgestattet ist und bei dem das Anschlußteil (19) um den Nadelausgang (11) des Behälters herum eine Rille (10) hat, um die Spitze (2a) der schräggestellten und zurückgedrückten Nadel aufzunehmen.

14. Set nach Anspruch 12 oder 13, bei dem das elastische Plättchen (21) durch eine Wand gebildet wird, die quer am proximalen Ende des Behälters angeordnet ist, die jedoch mit dem Behälter nur über einen Teil ihres Umfangs fest verbunden ist.

15. Set nach einem der Ansprüche 12 bis 14, bei dem der Behälter ein Ablenkermittel umfaßt, das durch eine Rampe (22) gebildet wird, die durch einen innenliegenden Höcker der Behälterseitenwand gebildet wird, und bei dem die Wand, die das elastische Plättchen (21) bildet, am Ende dieser Rampe als Spritzguß ausgeführt ist.

16. Set nach einem der Ansprüche 1 bis 15, bei dem der Kurzkatheter mit einem Eingangsanschlußteil (7) ausgestattet ist, das als Abstützung zum Drücken für den Anwender dient, und bei dem das distale Ende (3a) zum Anschluß des Behälters (3) an den Katheter für das Ankoppeln dieses Endes an das Anschlußteil angepaßt ist, und um in dieses Anschlußteil einzudringen.

17. Set nach Anspruch 16, bei dem das distale Ende des Behälters mit einem Anschlußteil (19) für seinen Anschluß an den Katheter versehen ist, wobei dieses Anschlußteil in das Anschlußteil des Katheters eindringt.
